# EUROPEAN PATENT APPLICATION

(11) **EP 1 369 123 A1**
(43) Date of publication of application: **10.12.2003**
(21) Application number: 03253287.1
(22) Date of filing: 24.05.2003
(51) Int. Cl.: A61K 35/78, A61P 3/10

(54) **A health-care product comprising lotus rhizome and process for its preparation**

(30) Priority: 07.06.2002 CN 02113847 U
(71) Applicant: Yunnan Baiyao Group Co., Ltd, Zone, Kunming (CH)
(72) Inventor: Pan, Ling, Kunming, Yunnan 650032 (CN); Li, Deliang, Kunming, Yunnan 650032 (CN); Zhang, Liqun, Kunming, Yunnan 650032 (CN); Tang, Wenxu, Kunming, Yunnan 650032 (CN); Yang, Chengjin, Kunming, Yunnan 650032 (CN); Gao, Chongkun, Kunming, Yunnan 650032 (CN); Feng, You, Kunming, Yunnan 650032 (CN); Chen, Mian, Kunming, Yunnan 650032 (CN)
(74) Representative: Browne, Robin Forsythe, Dr.

(57) **Abstract**

The present invention pertains to a multifunction health-care product and to a process for the preparation of the same. The health-care product comprises a powder of lotus root joints or an extract of the joints as main active ingredient. A preferred composition of the product comprises (part by weight): an extract of the joints 4 ∼ 7.5, a powder of lotus root joints 2 ∼ 15, green tea and/or extract thereof 0.08-2, notoginseng and/or extract thereof 0.08-0.5. The process for preparation of the composition comprises: directly pulverizing the lotus root joints, putting the pulverized lotus root joints in product-grade solvent for extraction, then filtering it, the filtrate solution is thus the extract of the joints. The product of the invention can effectively improve IR, thus improving and preventing type-II Diabetes Mellitus, hypertension, hyperlipoidemia and cardio-cerebral vascular diseases caused by being obesity. It also has the effect of losing weight.

## Description

### Technical field

The present invention pertains to a health-care product, especially to a health-care product capable of improving insulin resistance(IR) and to a process for its p reparation as well as its uses.

### Background of prior art

Along with the continuous improvement of living standard and the change of lifestyle, the number of people suffering from obesity, type-II Diabetes Mellitus, hypertension, hyerlipoidemia and cardio-cerebral vascular diseases have continuously increased, and these diseases often appear in complications. Own to high lethality and disability rate, these diseases represent important ones threatening human health at present. In recent decade, many domestic and foreign studies have indicated that, the the said diseases have the same pathogenetic basis, i.e. Insulin Resistance (referred to as IR). IR is the key cause of the said diseases, and obesity is the prelude of other diseases. IR means that the normal or higher insulin (Ins) level is contained in blood of human body, but it could just perform an insufficient biological effect, i.e., the Ins-sensitivity and -reactivity of the targeted tissue and organs (muscle, liver and fat tissue, et al) of organic Ins have being decreased. To overcome IR, B islet cells have to secret more Ins, thus cause compensative high hyperinsulinemia (HI), and HI could in turn further induce or aggravate the said diseases. At present, modern medicine has turned its affords to improve IR for a better treatment of the said diseases.

At present, the medicines for improving IR are mainly chemically synthesized substances (referred to as western medicine), such as insulin s ensitizer, Thiazolidine d iketone derivatives (TZD) for the treatment of type-II Diabetes Mellitus. However, the time in clinical application of this type of medicines is not long enough, the long-term adverse effect thereof still needs to be examined, so these medicines seem to be unsuiTable for administration for long time. Until now, there is still no instant Chinese herbal medicine or health-care product that has definite improvement and/or treatment effect on IR.

Since IR has complicated pathogenesis, which involves deficiency of different level and link in Ins pre-receptor, receptor and post-receptor. So far, there is still a strong demand for a safe, effective medicine that could comprehensively improve IR and the related diseases.

### Summary of the invention

To overcome the deficiency in existing technology, the present invention is directed to provide a multifunction health-care product, which could improve IR effectively, prevent the onset of type-II Diabetes Mellitus, hypertension, hyperlipoidemia and cardio-cerebral vascular disease caused by being obesity without adverse effect. As to the existing symptoms caused by the said diseases, they could also be substantially improved; Meanwhile, it also has a good anti-obesity effect.

### Detailed description of the invention

According to one aspect of the invention, there is provided a multifunction health-care product comprising a powder of lotus root joints or an extract of lotus root joints.

In one preferred embodiment of the health-care product, it is preferably to further comprise either green tea and/or its extract or notoginseng and/or its extract, or the mixture of the both. The composition of the preferred product thereof comprises: extract of lotus root joints 4 ~ 30 part by weight, more preferably 5~15 part by weight, most preferably 4 ~ 7.5 part by weight, green tea and/or its extract 0.08-2 part by weight, notoginseng and/or its extract 0.08-0.5 part by weight. In addition, it could further comprise 4~15 part by weight of a powder of lotus root joints.

The health-care product described above could be formulated into conventional administration form, such as Tablet, capsule, soluble granule, solution or injection, et al.

According to another aspect of the invention, there is provided a process for preparation of the health-care product comprising the following steps:
a) Pulverizing lotus root joints and thus obtaining a powder of the joints;
b) Putting the powder of the joints in a product-grade solvent for extraction, then filtering it, and using the filtrate solution as the extract of the joints; and
c) Blending conventional product-grade adjuvant with the powder of the joints or the extract of the joints, and then formulating the blend into a form of Tablet, capsule, soluble granule, solution or injection.

The said solvent is conventional food-grade solvent, among which fresh water and ethanol are preferred.

The steps a) and b) could be carried out by any of the following variants:
( 1 )Drying lotus root joints and pulverizing the dried joints into a powder having a granule size of 20 mesh; putting the powder into a container and adding therein 5 ~ 10 times by weight of 30% ~ 90% ethanol, based on the weight of the powder; extracting it at room temperature for 24 hours, then filtering the mixture; extracting the filtrate residue for another 2 times using the same procedure; combining the filtrate solutions, concentrating the combined filtrate solution under vacuum and freeze-drying it into a dried powder; or
(2) Drying the lotus root joints and pulverizing the dried joints into a powder having a granule size of 20 mesh, putting the powder into a container and adding therein 5 ~ 10 times by weight of 30% ~ 90% ethanol, based on the weight of the powder; heating it in water bath and refluxing for 30 ~ 60 minutes, then filtering it while it is hot; extracting the filtrate residue for another 2 times using the same procedure as mentioned above; combining the filtrate solutions, concentrating it at a low temperature under reduced pressure, and freeze-drying it into a dried powder; or
(3) Drying lotus root joints and pulverizing it into granules of soybean's size; putting it into a container and adding therein 6 ~ 10 times by weight of fresh water, based on the weight of the powder; heating it at its boiling point for 30 ~ 40 minutes, then filtering it while it is hot; extracting the filtrate residue for another 2 times using the same procedure as mentioned above; combining the filtrate solutions, concentrating the combined solution under vacuum and drying it into a powder.

Where a product in the form of a capsule is to be produced, it is preferred to further use 2 ~ 15 part by weight of a powder of lotus root joints in step b) described above, as powder of lotus root joints contains not only active substances, but could also act as an adjuvant required for capsule. The said green tea extract contains mainly tea polyphenol, notoginseng extract contains mainly full notoginseng saponin. In addition, a cinnamon naptha enveloped with β―cyclodextrin could also be blended into the composition of the capsule product, according to the invention.

According to an additional aspect of the invention, there are provided new uses of the inventive product in preparation of medicines for improving human insulin resistance; for treating or preventing obesity; for treating or preventing hypertension; for treating or preventing hyperlipoidemia; for treating or preventing Diabetes Mellitus; for improving blood viscosity, preventing thrombosis and promoting microcirculation; for treating or preventing Alzheimer's Disease and against senility.

The health-care product according to the invention is made from natural edible plant products, mainly from natural lotus root joints, and it thus takes the advantages of an easy availability and a cheaper price of the raw materials, a safety in administration of it in long term without adverse effect, in addition to its capability of effectively improving IR, preventing the onset of type-II Diabetes Mellitus, hypertension, hyperlipoidemia and cardio-cerebral vascular diseases caused by being obesity and a good anti-obesity effect.

### Brief description of Figures

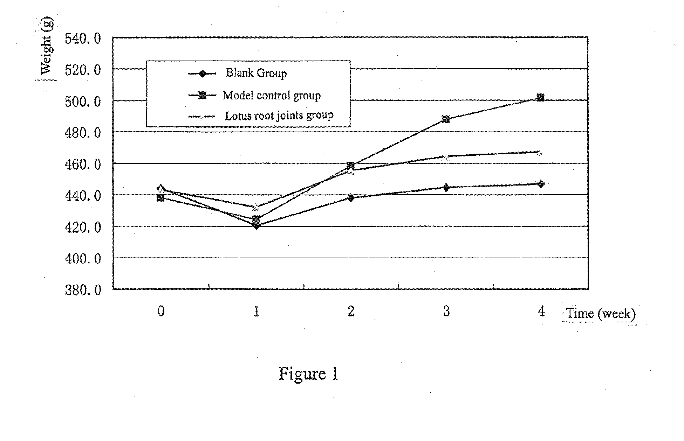

Figure 1 shows a trend of Change in weight of rats in different group for trial.

### Preferred embodiments of the Invention

For a better understanding in the essence of the present invention, the favorable medical effects of the product according to the invention are shown through systemic pharmacological test data and result as follows. However, they should not be understood as any restriction to the protection scope of the present invention that is defined by the appended claims.

### Example 1. The effect of lotus root joints on model of nutrition-type obesity rat

### 1.1 Material and test method

### 1.1.1 Preparation of experimental material and feed

### ( 1 ) Experimental material

Clean and dry the lotus root joints in ambient air, then put them in an oven and dry them in forced air under 65 □, and pulverize them into a fine powder having a granular size of less than 20 mesh. The powder is ready for use on that very day.

### ( 2 ) Positive medicine

The Ninghong anti-obesity tea (a product manufactured by Jiangxi Ninghong group, Trade No. 21101102) is pulverized into a fine powder for use on that very day.

### ( 3 ) Experimental feed

Concentrated high calorific feed: basal feed 40%, lard oil 35%, saccharose 15%, egg 8%, salt 2%.

Experimental feed: Take samples of the concentrated high calorific feed in certain amount and combine it respectively with certain amount of the basal feed, the powder of lotus root joints and the positive medicine powder, make them into model-type high calorific feed, lotus root joints-type high calorific feed and positive medicine-type high calorific feed using pelleter (the lotus root joints-type high calorific feed contains 68% concentrated high calorific feed, 32% the powder of lotus root joints, the model-type high calorific feed contains 32% basal feed in replacement of positive medicine powder, the 32% of the positive medicine powder in the positive medicine-type high calorific feed is consisted of 21% the basal feed and 11% fine powder of the Ninghong anti-obesity tea).

After the preparation of the feeds described above, desiccate them in ambient air, and process a new batch every 7 ~ 10 days.

### 1.1.2. Experimental animals and their group

SD male rats having weight of 444±17g, randomly assigned to: blank group (group □), model control group (group □), lotus root joints group (group □) and positive medicine group (group □). 8 animals for each group and are separately fed in single cage.

### 1.1.3. Model establishment and administration

Each rat is allowed to have 22g corresponding experimental feed at 5 p.m. everyday, i.e. basal feed 22g/animal for group □; model-type high colorific feed, lotus root joints-type high colorific feed and positive medicine-type high colorific feed 22/animal are given respectively to group □ ~ □ (every 22g of each feed contains 15g concentrated high colorific feed, the rest 7g are consisted respectively of 4.6g of basal feed, powder of lotus root joints and basal feed and 2.4g positive medicine powder). Each rat is allowed to have 8g basal feed the next morning, and no additional feed is additionally supplied, tap water is available ad libitum for rats in each group.

### 1.1.4. Observation indicator and method

### (1) General items

Including appearance of spirit, skin and hair, eyes, activity, eating, drinking and evacuation of the animal.

### ( 2) Intake amount

A form is designed and intake amounts are recorded every day using the form.

### ( 3 ) Weight taken once a week.

### ( 4 ) Glucose tolerance

Measured at the end of 4^{th} weeks after the beginning of experiment. Rats have been on fast for 6 hours, then take 6 of them, intraperitoneally inject 2g glucose/Kg animal, and sample blood through vena caudalis, and blood sugar is measured in 0, 0.5, 1 and 2 hours using rapid blood sugar calcimeter.

### ( 5 ) Blood sugar, serum TC, TG, HDL -c and Ins on Fast

At the end of experiment, sample blood from femoral vein, separate the serum, a part of it is submitted to the clinical laboratory in No. 1 hospital affiliated to Kunming medical college for test (by full automatic biochemical analyzer), and blood sugar, serum TC, TG, (color comparison in 7230 spectrophotometer) are manually measured by ourselves according to kit instruction.

### ( 6 ) Wet weight of celiac fat and testicle fat pad

Dissect the rat after blood sample, separate celiac fat and testicle fat pad, place them on the filtering paper to remove tissue fluid, their weight is taken by electronic scales and recorded.

### 1.1.5 Statistical method

Process the experimental data obtained with PEMS (Package for Encyclopaedia of Medical Statistics, edited by Public Health-Care College of Huaxi Medical University). Single factor analysis of variance and q-test are adopted for multi-group mean and pair-wise comparison.

### 1.2 Test result

### 1.2.1. GENERAL ITEMS

Except for the death of No. 1 rat in group □ and the poor uptake of positive medicine-type feed by rats in group □ (since rats in this group had never eaten up the positive medicine-type high colorific feed given to them, which cause it impossible to reflect the true effect of medicine, thus it was eliminated from the experiment), no abnormality was found in other rats.

### 1.2.2 The Change in weight of rats in each group at the end of 0 ~ 4^{th} week

See Table 1 ~ Table 5 and figure 1. Table 1 shows that, there was no difference in the weight of rat in each group before experiment, thus the weight of each group was comparable. It could be found that, at the end of 1^{st} week, there was no significant difference among weight of rats in each group, while the rat weight of each group had decreased at various degrees. It was considered to be caused mainly by the sudden change of living environment and lifestyle.

Table 3 shows that, at the end of 2^{nd} week, weight in model group and lotus root joints group are respectively 4.6% and 3.9% higher than that in blank group, but without significant difference. According to the amount of and percentage of weight gaining, model group had the largest gaining, which was significant higher than that in blank group ( P<0.05 ), lotus root joints group is less higher, but they had no significant difference from the blank group. This result indicated that the high colorific feed could increase the weight in adult rats having a about 400gbody weight, thus continuous feeding with this kind of feed could induce obesity in rats. Lotus root joints' preventive effect on excessive weight gaining induced by high colorific feed had not demonstrated here.

Table 4 shows that, at the end of 3^{rd} week, weight in model group was significantly higher than that in blank group (9.7% higher, p<0.01), while lotus root joints group had no significant difference from blank group (4.3% higher, p>0.05). According to the amplitude of weight gaining, model group had the largest one (p<0.01 compared with blank group), lotus root joints group was just slightly higher than blank group, but without significance. This result indicated that, up-taking this high colorific feed could cause the weight of adult rats of about 400g to be significantly heavier than that of rats at the same age that intake a common feed, and lotus root joints has relatively significant preventive effect on the excessive weight gaining caused by high colorific feed.

Table 5 shows that, at the end of 4^{th} week, weight in model group further exceeded that in blank group (12.3% higher ,p<0.01); compared with blank group ,the situation in lotus root joints group was similar to that in last week (4.5% higher, p>0.05), while compared with model group, their weight had been significantly reduced (p<0.01). The amplitude of weight gaining in each group was smaller than that before previous 2 weeks, while amplitude in model group was relatively larger. This result further indicated that, taking-up this high colorific feed could cause the weight of adult rats of about 400g to be significantly heavier than that of rats at the same age that intake a common feed, and lotus root joints had significant preventive effect on the excessive weight gaining caused by high colorific feed.

Figure 1 shows the trend of Change in weight in each group during the whole process of experiment from start to end.

It could be seen from figure 1 that, situation of lotus root joints group was relatively similar to that of blank group, especially in the late stage of experiment; model group demonstrated continuously higher weight than blank group. It indicated that this high colorific feed could cause progressively excessive weight gaining in adult rats of about 400g, and the weight at final stage of experiment was significantly heavier than rats at the same age that intake a common feed; lotus root joints had relatively significant preventive effect on this weight gaining trend.

### 1.2.3 Blood glucose( Glu ), blood insulin ( Ins )and insulin sensitivity index (ISI) of rats in each group(Table 6)

Table 6 shows that, model group had higher Glu, but without significance, when compared with blank group; Lotus root joints group had lower Glu. Except for lotus root joints group, blood Ins in each experimental group was significantly higher than that in blank group (p<0.01 for all groups), and ISI thereof was significantly lower than blank group (p<0.01 for all groups); ISI in lotus root joints group had a smaller difference from blank group (p<0.05), but it was significantly higher than the other experimental groups (p<0.01 for all groups). This result indicated that, this obesity model had produced hyperinsulinemia, with significant peripheral insulin resistance (IR); lotus root joints could prevent the hyperinsulinemia caused by high colorific product, and could significantly reduce peripheral IR.

### 1.2.4 The result of total cholesterol (TC), triglyceride (TG) and high density lipoprotein cholesterol (HDL-c) of rats in each group (Table 7)

Table 7 shows that, TC in model group was significantly higher than blank group( p<0.01 ), so it was also true in the other experimental groups( p<0.01 ), which had no significant difference from model group. TG in model group was also higher, but without significant difference from blank group; lotus root joints group had the lowest TG; HDL-c in lotus root joints group was significantly higher than that in blank group. This result indicated that, this obesity model had abnormal blood fat, which was demonstrated by significant increase of blood TC, and blood TG also had a trend to increase; lotus root joints could effectively prevent the increasing trend of blood TG Lotus root joints didn't have significant preventive effect on the increase of TC, but a little effect had been demonstrated. Lotus root joints had shown the effect to increase blood HDL-c.

### 1.2.5 Weight of intraperitoneal fat and fat index in each group (Table 8)

Table 8 shows that, weight of intraperitoneal fat and fat index in model group had a significant trend to increase; lotus root joints group has a opposite trend to that of model group.

### 1.3 Summary

### 1.3.1 Evaluation of obesity rat model

Usually, there are two methods for establishing obesity model: nutrition-type obesity model and hypothalamic-type obesity model. The former one adopts SD rats that had just past the breast, raise them with high nutritional feed (basal feed plus milk powder, egg, lard oil, vitamin AD and fresh soybean sprout, et al) for a period of time (45 days), their weight will significantly exceed that of rats at the same age fed with basal feed. This model adopts young rat, which are in flourishing growth period and need a lot of nutrition. Giving high nutrition feed rich in protein, fat and various vitamins, they will obviously grow faster, which is demonstrated by significant increase of weight, similar to the obesity case among children of human being. There is no detailed report in articles concerning the issue: whether the obesity is complicated with other pathophysiological changes, such as abnormal blood fat, IR or glucose tolerance, et al. Analyzed from the growth phase (flourishing growth and development period) and the nutrition formulation provided (rich and complete in nutrition, high colorific), it is estimated that at least the pathological change caused by being obesity in this obesity model would not be significant, which would be quite different from practical clinical situation observed from most obesity and overweight patient.

This experimental model also belongs to nutrition-type obesity model, the rats adopted weight about 444g having past their flourishing growth and development period, and equivalent to adult, the model feed adopted is basal feed plus abundant lard oil, saccharose and eggs, which contains high colorific but unbalanced nutrition, which was relatively similar to the dietary factor causing clinical obesity and related diseases such as type-II Diabetes Mellitus. The result of this experiment shows that, rat weight in basal feed group increased slowly, while rat weight in high colorific feed group progressively increased, and became significantly higher than that of rats in basal feed group in short time (28 days), the rats were complicated with significant higher cholestrol, triglyceride and hyperinsulinemia, ISI was significantly reduced, so was glucose tolerance; intraperitoneal fat and fat index also has a trend to increase. This result indicated that, this rat obesity model could simulate most clinical adult obesity, thus it could be used as a more suitable model for screening anti-obesity medicine.

### 1.3.2. Evaluation of lotus root joints' anti-obesity effect

In this experiment, lotus root joints was dried and pulverized into fine powder, blended it into high colorific feed to feed three groups of rats for comparison; meanwhile, rats fed with pure high colorific feed and basal feed were taken as the control. As a result, it was found that, the weight gaining in lotus root joints group was significantly less than that of model control group, but similar to that in blank group, and the final weight thereof is significantly lower than that of model control group, without significant difference from blank group. This result indicated that, lotus root joints had a preventive effect on obesity caused by high colorific product. Its effect on celiac fat tissue was similar to what described above.

Obesity is not the sole problem of weight- or fat gaining. Clinically, obesity patients often have metabolic disorders in sugar and fat, IR is an important pathophysiological basis for obesity, and also an important and harmful factor for obesity patients' susceptibility to type-II Diabetes Mellitus, hypertension, hyperlipoidemia and ischemic heart disease. Therefore, to inspect the anti-obesity effect of a medicine, it should not only take weight and fat as the indicator, but should also investigate the effect of medicine on the pathophysiological changes described above.

The result of this experiment shows that, serum Ins of rats in model control group had significantly increased, while ISI significantly decreased, thus significant amount of IR had been produced, with significant increase in serum TC; TG also showed a trend to increase; serum Ins in lotus root joints group was significantly lower than that of model group, while ISI thereof was significantly higher than that of model group, thus IR had been relieved; meanwhile, serum TG level was not high. In retrospect of its effect on weight, the increasing trend of weight in lotus root joints group became similar to that in blank group at late stage. The above results indicated that, lotus root joints had a comprehensive effect on the prevention of being obesity caused by high colorific product.

Summary: the high colorific feed of this formulation could cause obesity in adult rats with complications of abnormal blood fat and IR; lotus root joints could prevent the excessive weight gaining in rats caused by this high colorific feed, and had a function to reduce intraperitoneal fat; lotus root joints could also reduce IR in this obesity rat model and had a function to prevent increase of TG thereof. Lotus root joints containing relatively more starch had the best anti-obesity effect.

### Example 2. The effect of extract of lotus root joints on nutrition-type obesity rat

### 2.1 Material and method

### 2.1.1 Medicine and preparation thereof

Water decoction extract of lotus root joints : take proper amount of dry lotus root joints, pulverize it into granules of soybean's size, add it in 6 ~ 10 times by weight of fresh water, based on the weight of the granules; after it is heated to boiling point, continue to decoct it on small fire to keep boiling for 30 ~ 40 minutes, and then filter it while it is hot; extract the filtrate residue once more using the same method, and combine the extracted solutions; after being concentrated under reduced pressure and being frozen till to a powder having a water content of 6%, seal and store it for future use.

Ethanol refluxing extract of lotus root joints: take proper amount of dry lotus root joints, pulverize it into granules of 20 mesh, add it in 6 ~ 10 times of 30% ~ 90% by weight ethanol solutions, and heat it in water bath and refluxing for 1 hour, filter it while it is hot; Extract the filtrate residue once more using the same method, and combine the extracted solution; After concentrating it under reduced pressure, retrieve ethanol, freeze dry it in vacuum, the water content was 5%, seal and store it for future use.

Cold maceration extract of lotus root joints: take proper amount of dry lotus root joints, pulverize it into granules of 20 mesh, add it in 6 ~ 10 times of 30% ~ 90% by weight of ethanol solutions, and macerate it at room temperature for 24 hours; After filtration, add the ethanol solution at the same concentration described above to the filtrate residue for another 2 extractions, and combine the extracted solution; After concentrating it under reduced pressure, retrieve ethanol, freeze it in vacuum till to a powder having a water content of 5%, seal and store it for future use.

### 2.1.2 animal group, model establishment and administration of the product

SD male rats, weighed 434±17g were provided by animal laboratory of Yunnan Baiyao Group Co., Ltd. The animals were randomly assigned to: blank group, model group, water decoction extract group (water decoction group), ethanol refluxing extract group (ethanol refluxing group), ethanol cold maceration extract group (ethanol cold maceration group). 8 animals for each group, and were fed in separate cages. Model establishment was the same as that in experiment 1, i.e. give basal feed to blank group, high colorific feed to each of the other groups, dosage was the same as described before. Tap water was available ad libitum.

In the mean time of model establishment, administrate the animals with different feeds through gastric injection at 9 a.m. every day. The dosages of three extract powders described above were 2.4g/kg for water decoction group , 1.92g/kg for ethanol refluxing group , and 1.32g/kg for ethanol cold maceration group. All of them were diluted with distilled water of same volume before gastric injection. Distilled water of same volume was adopted for gastric injection in blank group and model group. The experimental period was 35days.

### 2.1.3 Observation indicator and method: identical to example 1.

### 2.1.4 Statistical method: identical to example 1.

### 2.2 Test result

### 2.2.1 General items

The animals' spirit, appetite, skin and hair, stool of rats in each group were all as usual during the whole process of experiment.

### 2.2.2 Effect of three lotus root joints extracts on the weight, Lee's index and fat index of adult nutrition-type obesity rats (Table 9)

Table 9 shows that, at the end of experiment, the rat's weight, Lee's index and fat index in model group were all significantly higher than those in blank group (p<0.01). Among the three test groups, the weights of animals in water decoction group and refluxing group were still heavier than that in blank group (p<0.01 for both), cold maceration group had no significant difference from blank group; compared with model group, weight in the three test groups were all significantly reduced (p<0.01); Lee's index in both water decoction group and refluxing group were higher than that in blank group (p<0.05), while cold maceration group had no significant difference from blank group; compared with model group, Lee's index in cold maceration group was relatively lower (p<0.01). None of the fat index in three test groups had significant difference from that in blank group, wherein the model group was the most similar to blank group. This result indicated that, all the lotus root joints water extract, lotus root joints ethanol refluxing extract and ethanol cold maceration extract had preventive effect on the excessive weight gaining and on increase of intraperitoneal fat in rats caused by high colorific feed, among which ethanol cold maceration extract seemed to have the best effect; lotus root joints water extract had comparable effect to that of lotus root joints ethanol refluxing extract.

### 2.2.3 The effect of three lotus root joints extracts on the blood sugar, insulin (Ins) and ISI of adult nutrition-type obesity rats (Table 10)

Table 10 shows that, the blood sugar in model group was higher than that in blank group (p<0.05), while the rest groups had no significant difference from blank group. Blood Ins in model group was significantly higher than that in blank group( p<0.01); Among three test groups, only water decoction group was slightly higher than blank group (p<0.05); compared with model group, blood Ins in all three test groups were reduced (p<0.05, p<0.01), with the cold maceration group being the lowest (p<0.01). ISI in model group was significantly reduced, among three test groups, ISI in both water decoction group and refluxing group were reduced, wherein the refluxing group was even lower (p<0.01), cold maceration group had no significant difference from blank group; Compared with model group, ISI in all the three test groups had increased to some extent, with the cold maceration group being increased more. This result indicated that, all the lotus root joints water extract, lotus root joints ethanol refluxing extract and ethanol cold maceration extract had preventive effect on increasing blood sugar and blood Ins in rats, and could improve IR, and ethanol cold maceration extract seemed to have the best effect, while lotus root joints water extract had comparable effect to that of lotus root joints ethanol refluxing extract.

### 2.2.4 The effect of three lotus root joints extracts on the serum TC, TG and HDL-c of adult nutrition-type obesity rats (Table 11)

Table 11 shows that, TC in model group had significantly increased (p<0.01), so did the three test groups (p<0.01); there was no significant decrease compared with model group. TG in model group had significantly increased (p<0.01), while increase in three test groups were not significant (p>0.05), and the cold maceration group was more similar to blank group. Had a lower HDL-c than blank group (p<0.05), the other three groups were all higher than that of model group, with cold maceration group was the highest (p<0.01), but all of the three groups had no significant difference from blank group. This result indicated that, lotus root joints water extract, lotus root joints ethanol refluxing extract and ethanol cold maceration extract had no significant preventive effect on increase of serum TC in rats caused by high colorific feed, but they did have preventive effect on the increase of serum TG and reduce of HDL-c thereof.

### 2.3 Summary

Example 2 repeated the model establishment method described in example 1, except that the period of conducting this example was prolonged for 1 week. The result showed that, the weight, Lee's index and intraperitoneal fat of the animals in this model had further exceeded than normal rats, and blood sugar after on fast was higher than that of rats in blank group, there was significant increase in blood Ins, significant reduce in ISI, significant increase in blood TC and TG, and reduce in HDL-c. All these results indicated that this nutritional rat model had significantly excellent repeatability, it was a relatively ideal animal model for the study of obesity, IR and related pathophysiological disorders.

According to the experience and habit in clinical administration, example 2 adopted several conventional extraction methods, i.e. using water decoction, 65% ethanol thermal refluxing, and 65% ethanol cold maceration to extract the total active ingredients in lotus root joints. Then orally administrate the frozen powder prepared from the extract to rats and compare the clinical effect. A control group was established using original medicinal powder. The result demonstrated that, all the three extracts had preventive effect on the excessive weight gaining, on increase of Lee's index and intraperitoneal fat, meanwhile, they had some preventive effect on the increase of blood sugar after on fast and on blood Ins, and could improve IR, some preventive effect was also presented on the increase of TG and on decrease of HDL-c; as to the increase of TC, the preventive effect of these three extracts was not significant which was similar to that of raw lotus root joints powder.

### Example 3. Effect of composition containing lotus root joints on nutrition-type obesity rats

### 3.1 material and method

### 3.1.1 Medicine

□ freeze dry powder of cold maceration extract of lotus root joints (its preparation was identical to that recorded in experiment 2) ;
□ notoginseng extract (notoginseng total saponin : content □ 88% , Rb1 > 30% , Rg1>20%; notoginseng leaf saponin : content of saponin element □32%, calculated as original ginseng diol) ; □ green tea extract(tea polyphenol□40% catechin□25%, EGCG□25% , caffeine < 5 %=

### 3.1.2 Animal group and model establishment administration

SD male rats, weighed 435±16g, were provided by animal laboratory of Yunnan Baiyao Group Co., Ltd. Animals were randomly assigned to: blank group, model group, lotus root joints ethanol cold maceration extract freeze dry powder group (lotus root joints simple prescription group), lotus root joints ethanol cold maceration extract freeze dry powder plus notoginseng extract group (lotus root joints compound prescription group 1), lotus root joints ethanol cold maceration extract freeze dry powder plus green tea extract group (lotus root joints compound prescription group 2) and lotus root joints ethanol cold maceration extract freeze dry powder plus notoginseng extract plus green tea extract group (lotus root joints compound prescription group 3). 8 animals were in each group, which were fed in separate cages. Give basal feed to blank group, high colorific feed to each of the other groups to establish model (the method was identical to that in previous two experiments). Tap water was available ad libitum for each rat.

In the mean time of model establishment, administrate the feed through gastric injection at 9 a.m. every day: 1.32g/Kg lotus root joints cold maceration extract freeze dry powder for lotus root joints simple prescription group; 1.32g/Kg lotus root joints cold maceration extract freeze dry powder + 0.066g/Kg notoginseng saponin and 0.066g/Kg notoginseng leaf saponin for lotus root joints compound prescription group 1; 1.32g/Kg lotus root joints cold maceration extract freeze dry powder + 0.132g/Kg tea polyphenol for lotus root joints compound prescription group 2; 1.32g/Kg lotus root joints cold maceration extract freeze dry powder + 0.066g/Kg notoginseng saponin and 0.066g/Kg notoginseng leaf saponin + 0.132g/Kg tea polyphenol . Each medicine described above was diluted with distilled water of the same volume before gastric injection. Distilled water of the same volume was adopted for gastric injection in blank group and model group. Administration of the feeds continued for 28days.

### 3.1.3 Observation indicator and method

### General Items: Animal's spirit, appetite, skin and hair, activity and stool

Body weight, length and Lee's index: weight was taken once a week; length was measured at the end of experiment (the length from nose tip to the anal of rat, expressed in cm). Lee's index = weight (g)/body length (cm2).

Glucose tolerance: After on fast for 6 hours, rats were intraperitoneally injected with 2g glucose/Kg, and blood sugar was measured at 0, 30, 60 and 120 minutes.

Blood sugar after on fast, Ins and blood fat (including TC, TG, LDL-c and HDL-c), calculation of arteriosclerosis index AI (AI=TC/HDL-c) and Ratio of Coronary Heart Disease R-CHR (R-CHR=LDL/HDL-c).

### Fat weight in body: Intraperitoneal fat, et al, converted to celiac fat g/100g weight

The size and amount of fat cell: Take a small piece of fat from the same area around genitals, fix it in 2.5% formaldehyde-ethanol solution, slice it in paraffin, count the fat cells in a full visual field under 400xmicroscope, and measure the size of fat cells with micrometer.

### Serum SOD and LPO

### Blood viscosity

### 3.1.4 Statistical method: identical to example 1.

### 3.2 Test result

### 3.2.1 General items: The general items of rats in each groups were good during the whole process of experiment, no abnormality was found.

### 3.2.2 The effect of lotus root joints compound prescription on the weight, Lee's index of nutrition-type obesity rats(Table 12)

Table 12 shows that, at the beginning of experiment, there was no difference in rats' weight among all groups. At the end of experiment, the rats' weight in model group was significantly higher than that in blank group ( p<0.01 ); Among four test groups, the simple prescription group, compound prescription group 1, compound prescription group 2 also had weight higher than blank group (p<0.01,P<0.05), the amplitude in compound prescription group 2 was relatively smaller (P<0.05), compound prescription group 3 had no significant difference from blank group. Compared with model group, weight in four test groups were all significantly reduced (p<0.01); compared with single prescription group, only weight in compound prescription group 3 was lower (p<0.05). There was no significant difference among body length of the animal in each group. Lee's index in model group was significantly higher than that in blank group (p<0.01); while four test groups had no significant difference from blank group; compared with model group, Lee's index in four test groups were relatively lower (p<0.01), in which compound prescription group 3 had the lowest. This result indicated that, all these four formulations had preventive effect on the excessive weight gaining and increase of Lee's index in rats caused by high colorific feed, and the compound prescription 3 had the best effect, then is compound prescription 2; compound prescription 1 had a comparable effect to that of simple prescription.

### 3.2.3 The effect of lotus root joints composition on the body fat and fat cells of nutrition-type obesity rats (Table 13)

Table 13 shows that, the celiac fat of rats in model group was significantly more than that in blank group (p<0.01); Among the four test groups, only simple prescription group and compound prescription group 1 were higher than blank group (P<0.05); compound prescription group 3 was most similar to blank group. Fat cells of rats in model group were significantly less than that in blank group (p<0.01); Among four test groups, only simple prescription group and compound prescription group 1 were lower than blank group (P<0.05, p<0.01); compared with model group, all the four test group had relatively more fat cells; compared with simple prescription group, compound prescription group 2 and compound prescription group 3 had more fat cells (P<0.05, p<0.01), with compound prescription group 3 having the most and being most similar to blank group. A fat cell diameter of rats in model group was significantly larger than that in blank group ( p<0.01 ); Four test groups were also larger than that in blank group, but the difference between compound prescription group 2 and compound prescription group 3 was relatively smaller (p<0.05); All the four test group had relatively smaller fat cell diameter than that in model group (p<0.01); compound prescription group 3 had a significantly smaller diameter than simple prescription group (P<0.01). This result indicated that, all these four formulations had preventive effect on the increase of celiac fat, on fat cell hypertrophy and on the decrease of fat cells caused by high colorific feed, and the compound prescription 3 had the best effect, then was compound prescription 2, while compound prescription 1 and simple prescription had comparable effect.

### 3.2.4 The effect of lotus root joints compound prescription on the blood fat of nutrition-type obesity rats(Table 14)

Table 14 shows that, the serum TC of rats in model group was significantly elevated ( p<0.01 ) ; Among the four test groups, simple prescription group was still significantly higher than blank group (P<0.05), compound prescription group 1 and compound prescription group 2 were slightly higher than blank group (p<0.05), compound prescription group 3 had no significant difference from blank group; all the three compound prescription groups were lower than model group (p<0.05, p<0.01). Serum TG in model group was also elevated (p<0.05); All the four test groups were lower than model group (p<0.05), with no significant difference from blank group. LDL-c in model group was significantly elevated (p<0.05), so was in simple prescription group (p<0.01), without increase in other three test groups. HDL-c in model group was reduced (p<0.05); all the four test groups were higher than model group (P<0.05), with no significant difference from blank group. This result indicated that, lotus root joints simple prescription didn't have had significant preventive effect on the increase of serum TC of rats caused by high colorific feed, while compound prescription 1 and compound prescription 2 had some effect, and compound prescription 3 had the best effect. All these four formulations had preventive effect on the increase of serum TG in this model. Lotus root joints simple prescription didn't have significant preventive effect on the increase of serum LDL-c in this model, while three compound prescriptions did. All these four formulations could prevent the decrease of serum HDL-c in this model.

### 3.2.5 The effect of lotus root joints compound prescription on the arteriosclerosis index (AI) and Ratio of coronary heart disease ( R-CHD ) in nutrition-type obesity rats(Table 15)

Table 15 shows that, AI of rats in model group was significantly elevated ( p<0.01 ); All the four test groups had no significant difference from blank group, wherein compound prescription group 1 and compound prescription group 3 were lower than model group and more similar to blank group. R-CHD in model group was significantly higher than that in blank group (p<0.01), among four test groups, simple prescription group was still significantly higher than blank group (p<0.01), compound prescription group 2 was slightly higher than blank group (p<0.05); all the four test groups were significantly lower than model group (P<0.05), with three test groups being significantly lower than simple prescription group (P<0.05, P<0.01), and compound prescription group 3 was the lowest (p<0.01). This result indicated that, All these four formulations had preventive effect on the increase of AI and R-CHD in this model, wherein compound prescription had the best result, then was compound prescription 1, and followed by compound prescription 2, with simple prescription being left behind.

### 3.2.6 The effect of lotus root joints compound prescription on the glucose tolerance in nutrition-type obesity rats (Table 16)

Table 16 shows that, among the fasted blood sugar of each group, model group was relatively higher, but without significant difference. At each time point after glucose load, blood sugar in model group had been significantly elevated; blood sugar of simple prescription group, compound prescription group 1 and compound prescription group 2 were higher than blank group at each time interval after glucose load, only compound prescription group 3 was similar to blank group. But compared with model group, blood sugar of each group at each time point after glucose load was lower (p<0.01 for all). At each time interval after glucose load, blood sugar in compound prescription group 3 was lower than that in single prescription group (p<0.01), compound prescription group 1 and compound prescription group 2 were lower than single prescription group (p<0.05) at 120minutes after glucose load. This result indicated that, All these four formulations had certain preventive effect on the decrease of glucose tolerance in rats caused by high colorific feed, among which compound prescription 3 had the best effect, compound prescription 1 and compound prescription 2 had obvious effect, while simple prescription had relatively weak effect.

### 3.2.7 The effect of lotus root joints compound prescription on the blood sugar, Ins and ISI in nutrition-type obesity rats (Table 17)

Table 17 shows that, there was no significant difference among the fasted blood sugar of each group, while fasted serum Ins in model group had significantly increased (P<0.01); All the four test groups had significantly lower serum Ins than model group (P<0.01), with no significant difference from blank group. Ins in model group was significantly lower than that in blank group ( P<0.01 ); Among four test groups, simple prescription group, compound prescription group 1 and compound prescription group 2 were still lower than blank group ( P<0.05 ) , compound prescription group 3 was similar to blank group; All the four test groups were significantly higher than model group. This result indicated that, All these four formulations could prevent the increase of serum Ins caused by high colorific feed, thus reduce the decrease of body's sensitivity to insulin, and compound prescription 3 had the best effect, while the other three prescriptions had similar effect.

### 3.2.8 The effect of lotus root joints compound prescription on the blood viscosity in nutrition-type obesity rats(Table 18).

Table 18 shows that, serum viscosity in model group had significantly increased (P<0.01); All the four test groups had lower serum viscosity than model group, in which three compound prescription groups were even lower (P<0.01); simple prescription group and compound prescription group 2 were still higher than blank group (P<0.01, P<0.05); both compound prescription group 1 and compound prescription group 3 were significantly lower than simple prescription group. Hct in model group was larger than that in blank group (P<0.05), while four test groups had no significant difference from blank group, wherein compound prescription group 3 was most similar to blank group. This result indicated that, all these four formulations had preventive effect on the increase of serum viscosity in this model, wherein compound prescription 3 and compound prescription 1 had the best effect, then was compound prescription 2, while simple prescription had a relatively weak effect. The situations were similar with regard to their effect on Hct.

### 3.2.9 The effect of lotus root joints compound prescription on SOD and LPO in nutrition-type obesity rats(Table 19).

Table 19 shows that, serum SOD in model group had significantly decreased (P<0.01), with no significant decrease in four test groups, in which compound prescription group 3 and compound prescription group 2 were more similar to blank group. LPO in model group had significantly increased (P<0.01); All the four test groups were significantly lower than that of model group (P<0.01) ; Except that simple prescription group was still higher than blank group (P<0.01), all the three compound prescription groups had no significant difference from blank group, and compound prescription group 3 was most similar to blank group, then was compound prescription group 2; all the three compound prescription groups were lower than simple prescription group ( P<0.05,p<0.01 ) , in which compound prescription group 2 and compound prescription group 3 were even lower ( p<0.01 ) . This result indicated that, all these four formulations had preventive effect on the increase of serum SOD and LPO in this model, wherein compound prescription 3 and compound prescription 2 had the best effect, then was compound prescription 1, while simple prescription had a relatively weak effect.

### 3.3 Summary

Example 3 adopted the adult nutrition-type obesity rat model which had been proven to have good repeatability. Investigations had been made on the effect of pure lotus root joints product, lotus root joints combined with extracted total notoginseng saponin and green tea extract tea polyphenol on many items of animals in this model such as weight, celiac fat, fat cell's size and number, glucose tolerance, IR, blood fat, blood viscosity and LPO, et al. The result showed that, using the pure lotus root joints product had caused certain improvement on items described above except serum TC, but its effect on celiac fat reduction, blood fat adjustment, blood viscosity improvement, anti-arteriosclerosis, anti-oxidization was still weak. In combination with total notoginseng saponin, it could cause more significant decrease in serum TC and blood viscosity, and cause quite smaller AI and R-CHD; in combination with tea polyphenol, it could cause the amount of celiac fat and the number and size of fat cells to be closerer to normal value, and could increase the activity of SOD, reduce LPO. If it is combined with notoginseng total saponin and tea polyphenol, then all the above items would tend to become normal.

Notoginseng and one of its main effective active ingredient, notoginseng total saponin, is a Chinese traditional medicine and natural chemical substance under extensive study. Many studies have shown that, notoginseng saponin and notoginseng leaf saponin have good effect on blood fat adjustment, blood viscosity reduction and protection of vascular endothelial function. Obesity patients often show a complication of hyperlipoidemia and increased blood viscosity, with significantly higher incidence of arteriosclerosis than persons without obesity. This experiment adopted lotus root joints in combination with notoginseng saponin and notoginseng leaf saponin, the result indicated that the blood fat and blood viscosity in this obesity model tend to become normal, thus indicated that this combination was necessary for the comprehensive treatment of obesity.

Green tea is an herbal drug/food double function product that has been used for weight and fat reduction since thousands year ago; one of its main active ingredient, tea polyphenol, is also a kind of natural chemical substance under extensive study. Studies have shown that, tea polyphenol has significant inhibiting effect on accumulation of fat in the body and liver of rats which had been raised with fat-rich feed, and it could reduce TC in blood; At the same time, tea phenol also has excellent anti-oxidization effect, it could inhibit the oxidization of cholesterol through self-oxidization, thus reduce the deposition of cholesterol on artery wall. This adopted lotus root joints in combination with tea polyphenol had brought about the result: celiac fat in this model had significantly decreased, with significant decrease in fat cell's size, at the same time, blood SOD had significantly increased, with significant reduction of LPO, thus could lead to a conclusion that this composition had a comprehensive and good effect on obesity and the effect was also better than the single use of lotus root joints.

Compound prescription 3 in this experiment adopted lotus root joints extract as the principal drug to be combined with saponin from root, stem and leaf of notoginseng and with green tea polyphenol, the result showed that, this combination had integrated the advantages of these three substances, this model had achieved a relatively ideal effect in every respect. It was indicated that lotus root joints, as the principle drug, in combination with saponin from root, stem and leaf of notoginseng and with green tea polyphenol was a relatively ideal combination for the treatment of obesity.

Obesity is a complicated disease with various conditions, it is not only a matter of gaining in weight and fat, but, more important, obesity patients have significant IR and glucose, fat metabolic disorders, it is the very pathophysiological disorders that complicate obesity patients with other serious diseases such as type-II Diabetes Mellitus, abnormal blood fat, hypertension and arteriosclerosis, and cause the average lifetime of obesity population shorter than that of non-obesity population. This new combination according to the invention uses lotus root joints as the principal active ingredient, it not only has good anti-obesity effect, but more important, it has an outstanding effect on improving IR significantly. IR is the pathophysiological basis for obesity patient to have metabolic disorders in glucose, fat and to be susceptible for the said serious diseases, therefore, lotus root joints products, such as its powder or its extract, used as the principal active ingredient in the prescription, have irreplaceably important effect on the treatment and prevention of obesity. A combination with tea polyphenol and notoginseng saponin obtained from root, stem, leaf makes the prescription more effective, act more quickly for cleaning unwanted fat within body, for adjustment of blood fat, improvement of blood viscosity and protection of arterial wall, et al, and makes the prescription capable of not only hitting the key point of obesity, i. e., IR, enabling a thorough treatment to the basic grounds causing obesity, but also taking the advantage of combination of various positive effects on various pathophysiological disorders in obesity. Thus, the product of the invention has better effect not only on obesity itself, but also on the treatment and prevention of various complications thereof.

### Beneficial effect:

In one word, the present invention represents a substantial breakthrough in development of a safe and effective health-care product that could comprehensively improve IR and the related diseases through extensive and systemic study. The present invention adopts natural lotus root joints as the principal raw material, which was abundant in source, without adverse effect, of high quality and inexpensive, and readily acceptable to people, thus it could benefit millions of patients. The lotus root joints composition according to the invention in combination with tea polyphenol and notoginseng root-, stem- and leaf saponin, makes the prescription more effective, acts more quickly for cleaning unwanted fat within body, for adjustment of blood fat, for improvement of blood viscosity and for protection of arterial wall, et al; the product of the invention has not only a good therapy effect on the key link of being obesity, i.e., IR, enabling an effective and thorough treatment of it, but could also take the advantage of combination of various positive effects of the various active ingredients, taking account of various complicated pathophysiological disorders caused from being obesity. Thus, this combination has surprising effect not only on obesity itself, but also on the treatment and prevention of type-II Diabetes Mellitus, hypertension, hyperlipoidemia and cardio-cerebral vascular diseases induced by it.

**Table 1.**

| Weight of rats in each group at 0^{th} week | | |
|---|---|---|
| X±SD | | |
| Group | Number of animals n | Body weight (g) |
| Blank Group | 8 | 444.0±13.1 |
| Model control group | 7 | 442.0+20.8 |
| Lotus root joints group | 8 | 443.0±19.0 |
| p>0.05 for all | | |

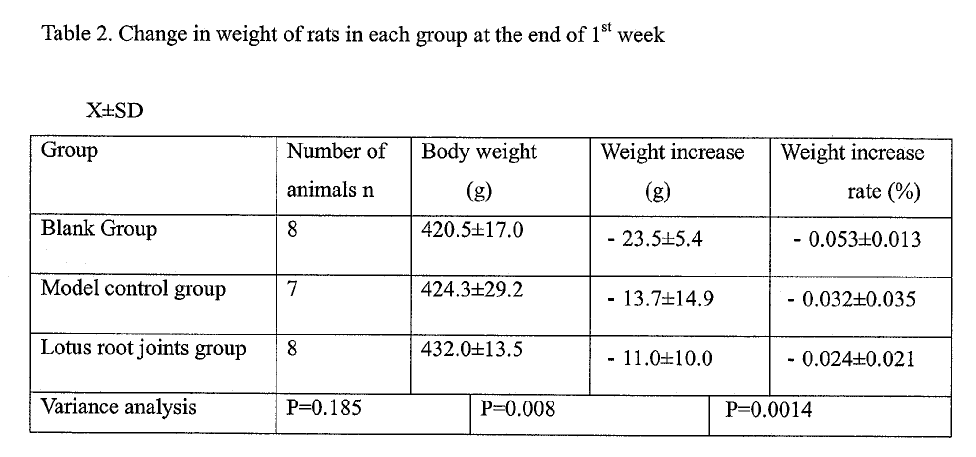

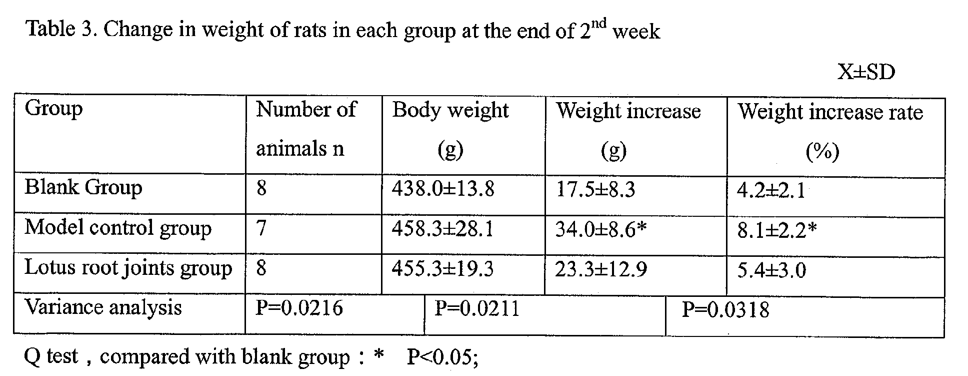

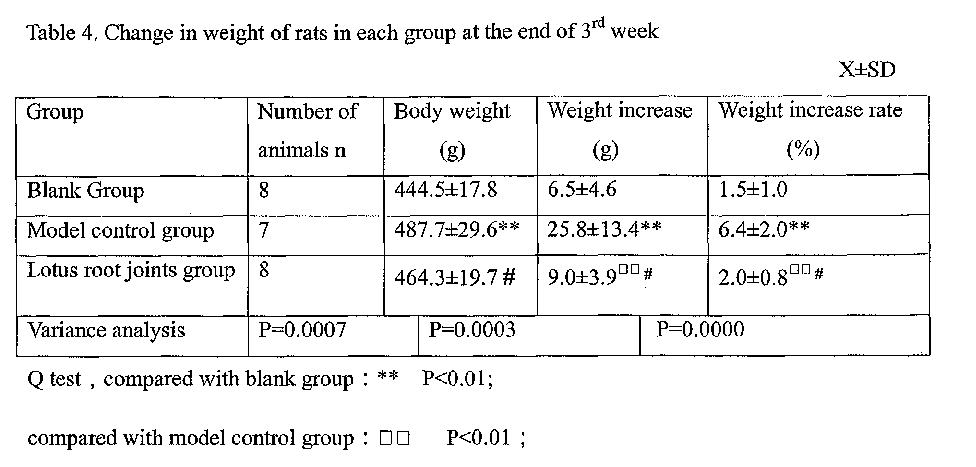

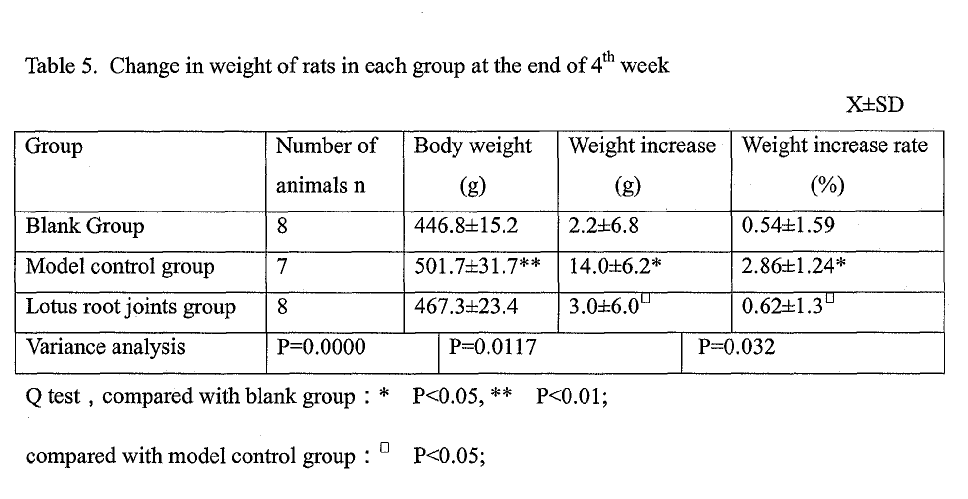

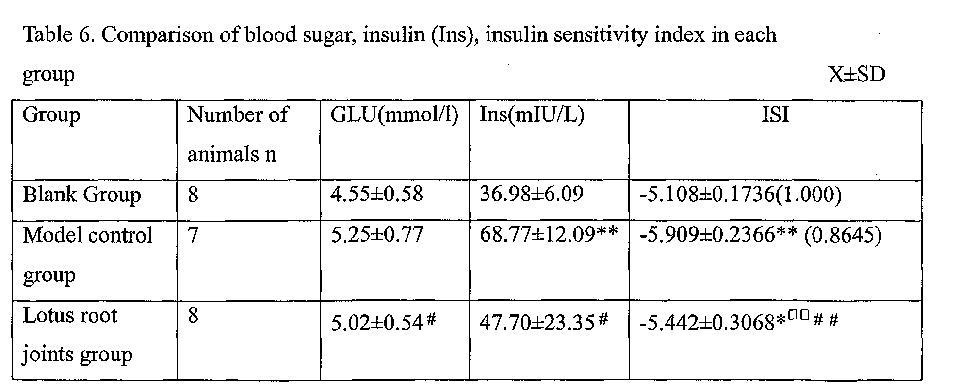

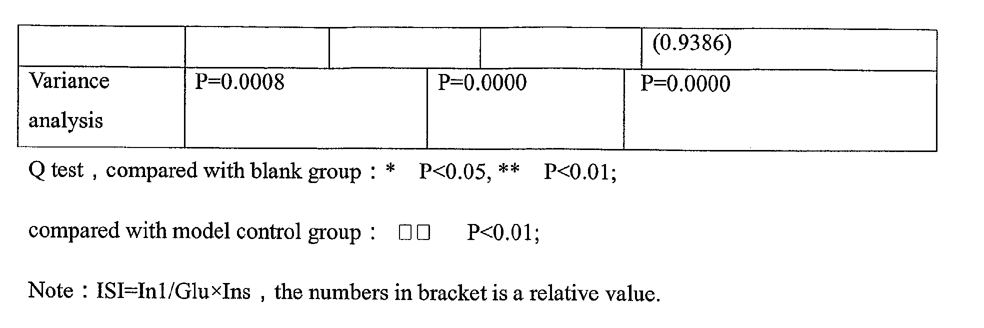

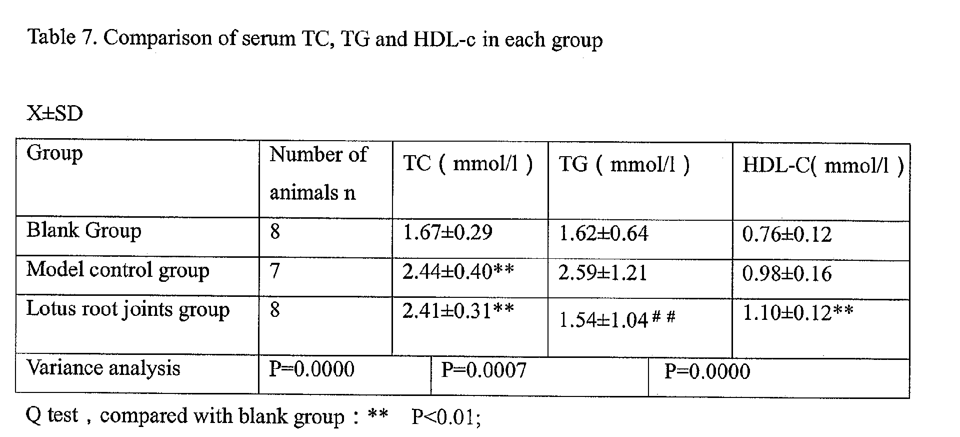

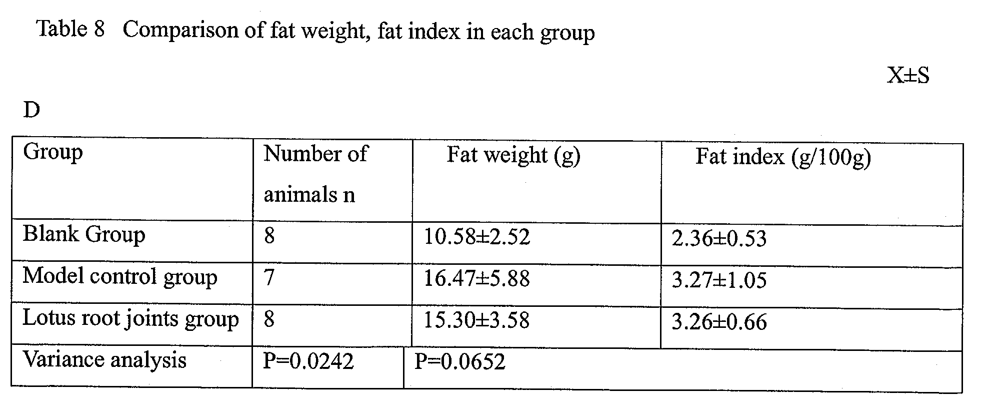

**Table 9.**

| Effect of three lotus root joints extracts on the weight, Lee's index and the fat index of adult nutrition-type obesity rats | | | | | |
|---|---|---|---|---|---|
| X±S | | | | | |
| Group | Init. Weight (g) | End weight (g) | Body length (cm) | Lee's index (g/cm²) | Fat rate (g/kg) |
| Blank group | 434.5±22.5 | 461.4±25.6 | 25.7±1.3 | 0.68±0.02 | 5.78±1.79 |
| Model group | 434.3±19.3 | 574.2±30.7** | 26.4±2.1 | 0.82±0.08** | 9.87±2.24** |
| Water decoction group | 434.8±21.2 | 530.3±30.1**^{□□} | 26.3±1.7 | 0.77±0.04* | 8.18±2.01 |
| Ethanol refluxing group | 433.9±15.1 | 528.3±29.4**^{□□} | 26.3±1.2 | 0.76±0.06* | 7.92±1.89 |
| Ethanol cold maceration group | 434.7±17.2 | 489.7±30.0^{□□} | 26.2±1.7 | 0.71±0.05^{□□} | 6.77±1.54^{□} |

| | | | | | |
|---|---|---|---|---|---|
| Compared with normal group : * p<0.05, | | | | | |
| ** p<0.01; | | | | | |
| compared with model group : □ p<0.05, | | | | | |
| □ □ p<0.01_{°} n=8 | | | | | |

**Table 10.**

| Effect of three lotus root joints extracts on the blood sugar, insulin (Ins) and ISI of adult nutrition-type obesity rats | | | |
|---|---|---|---|
| X±S | | | |
| Group | Blood sugar (mmol/L) | Ins(mmol/L) | ISI |
| Blank group | 4.62±0.51 | 36.78±5.89 | - 5.106±0.173 |
| Model group | 5.83±0.69* | 70.14±12.13** | - 6.013±0.324** |
| Water decoction group | 5.33±0.84 | 56.34±13.91*^{□} | - 5.691±0.412**^{□} |
| Ethanol refluxing group | 5.28±0.76 | 51.48±15.74^{□} | - 5.604±0.293*^{□} |
| Ethanol cold maceration group | 5.02±0.54 | 45.10±14.09^{□□} | - 5.416±0.231^{□□} |

| | | | |
|---|---|---|---|
| Compared with normal group : * p<0.05, | | | |
| ** p<0.01; | | | |
| compared with model group : □ p<0.05, | | | |
| □□ p<0.01_{°} n=8 | | | |

**Table 11.**

| Effect of three lotus root joints extract on the serum TC, TG and HDL-c of adult nutrition-type obesity rats | | | |
|---|---|---|---|
| X±S | | | |
| Group | TC ( mmol/L ) | TG(mmol/L) | HDL-c(mmol/L) |
| Blank group | 1.66±0.30 | 1.04±0.70 | 1.01±0.13 |
| Model group | 2.58±0.41** | 2.84±1.02** | 0.74±0.15* |
| Water decoction group | 2.41±0.32** | 2.43±1.07 | 0.98±0.29^{□} |
| Ethanol refluxing group | 2.43±0.54** | 1.98±1.12 | 1.06±0.24^{□} |
| Ethanol cold maceration group | 2.31±0.47** | 1.68±1.04 | 1.22±0.26^{□□} |

| | | | |
|---|---|---|---|
| Compared with normal group : * p<0.05, | | | |
| ** p<0.01; | | | |
| compared with model group : □ p<0.05, | | | |
| □ □ p<0.01_{°} n=8 | | | |

**Table 12.**

| Effect of lotus root joints composition on the weight, Lee's index of adult nutrition-type obesity rats | | | | |
|---|---|---|---|---|
| X±S | | | | |
| Group | Initial weight (g) | End weight (g) | Body length (cm) | Lee's index (g/cm2) |
| Blank group | 436.5±16.2 | 458.3±21.0 | 25.5±1.2 | 0.69±0.03 |
| Model group | 436.2±18.1 | 569.7±28.3** | 26.1±2.0 | 0.89±0.05** |
| Simple prescription group | 436.8±16.9 | 513.6±33.4**^{□□} | 26.1±1.8 | 0.75±0.07^{□□} |
| Compound prescription 1 group | 436.3±15.3 | 517.2±30.0**^{□□} | 26.2±1.3 | 0.74±0.05^{□□} |
| Compound prescription 2 group | 436.4±17.2 | 503.2±31.2*^{□□} | 26.1±1.4 | 0.73±0.06^{□□} |
| Compound prescription 3 group | 436.5±16.2 | 458.3±21.0 | 25.5±1.2 | 0.69±0.03 |

| | | | | |
|---|---|---|---|---|
| Compared with normal group : * p<0.05, | | | | |
| ** p<0.01; | | | | |
| compared with model group : □□ p<0.01 ; compared with simple prescription group : # p<0.05; n=7 | | | | |

**Table 13.**

| Effect of lotus root joints compound prescription on the body fat and fat cells of nutrition-type obesity rats | | | |
|---|---|---|---|
| X±S | | | |
| Group | Celiac fat (g/kg) | Fat cell (number/HPF) | Fat cell size (µm) |
| Blank group | 5.27±1.83 | 126.3±11.5 | 27.1±1.8 |
| Model group | 9.52±2.11** | 85.4±9.1** | 44.3±2.5** |
| Simple prescription group | 8.14±1.62* | 106.5+10.4*^{□□} | 36.5±2.1**^{□□} |
| Compound prescription 1 group | 7.83±2.23* | 98.2±8.3**^{□} | 38.1±3.4**^{□□} |
| Compound prescription 2 group | 7.07±1.64 | 112.4±11.5^{□□}^{#} | 34.2±3.3*^{□□} |
| Compound prescription 3 group | 6.06±1.58^{□} | 118.5±13.2^{□□}^{##} | 31.02.4*^{□□}^{##} |

| | | | |
|---|---|---|---|
| Compared with normal group : * p<0.05, | | | |
| ** p<0.01; | | | |
| compared with model group : □ p<0.05, | | | |
| □□ p<0.01; | | | |
| compared with simple prescription group : # p<0.05, | | | |
| # # p<0.01; n=7 | | | |

**Table 14.**

| Effect of lotus root joints compound prescription on the blood fat of nutrition-type obesity rats | | | | |
|---|---|---|---|---|
| X±S | | | | |
| Group | TC (mmol/L) | TG (mmol/L) | LDL-c (mmol/L) | HDL-c(mmol/L) |
| Blank group | 1.54±0.25 | 0.92±0.64 | 2.43±0.41 | 1.32±0.15 |
| Model group | 2.73±0.41** | 2.68±1.02* | 5.16±1.33** | 1.08±0.18* |
| Simple prescription group | 2.45±0.32** | 1.34±1.13^{□} | 4.97±1.47** | 1.37±0.09^{□} |
| Compound prescription 1 group | 2.09±0.40*^{□} | 1.20±0.89^{□} | 3.55±1.33 | 1.32±0.20^{□} |
| Compound prescription 2 group | 2.14±0.46*^{□} | 1.24±0.88^{□} | 3.87±1.54 | 1.28±0.19^{□} |
| Compound prescription 3 group | 1.93±0.52^{□□} | 1.01±0.66^{□} | 3.16±1.39 | 1.35±0.23^{□} |

| | | | | |
|---|---|---|---|---|
| Compared with normal group : * p<0.05, | | | | |
| ** p<0.01; | | | | |
| compared with model group : □ p<0.05, | | | | |
| □□ p<0.01 . n=7 | | | | |

**Table 15.**

| Effect of lotus root joints compound prescription on the AI and R-CHD of nutrition-type obesity rats | | |
|---|---|---|
| X±S | | |
| Group | AI | R-CHD |
| Blank group | 1.17±0.37 | 1.83±0.34 |
| Model group | 2.53±0.75** | 4.76±0.59** |
| Simple prescription group | 1.77±0.81 | 3.62±0.70**^{□□} |
| Compound prescription 1 group | 1.48±0.69^{□} | 2.65±0.66^{□□}^{#} |
| Compound prescription 2 group | 1.56±0.72 | 2.81±0.54*^{□□}^{#} |
| Compound prescription 3 group | 1.40±0.58^{□} | 2.42±0.45^{□□}^{##} |

| | | |
|---|---|---|
| Compared with normal group : * p<0.05, | | |
| ** p<0.01; | | |
| compared with model group : □ p<0.05, | | |
| □□ p<0.01 ; | | |
| compared with simple prescription group : # p<0.05, | | |
| ## p<0.01 . n=7 | | |

**Table 16.**

| Effect of lotus root joints compound prescription on the glucose tolerance of nutrition-type obesity rats | | | | |
|---|---|---|---|---|
| X±S | | | | |
| Group | Blood sugar (mmol/L) | | | |
| Blank group | 0 | 30 | 60 | 120(min) |
| Model group | 5.06±0.44 | 11.21±0.42 | 9.62±0.76 | 5.64±0.83 |
| Simple prescription group | 6.02±0.86 | 15.97±1.06** | 16.34±1.03** | 11.74±0.94** |
| Compound prescription 1 group | 5.33±0.49 | 14.02±0.97**^{□□} | 14.11±1.01**^{□□} | 9.83±1.12**^{□□} |
| Compound prescription 2 group | 5.41±0.91 | 13.98±1.04**^{□□} | 12.76±1.21**^{□□} | 8.01±0.98**^{□□}^{#} |
| Compound prescription 3 group | 5.30±0.56 | 14.11±1.15**^{□□} | 11.22±1.19**^{□□} | 7.88±1.02**^{□□}^{#} |

| | | | | |
|---|---|---|---|---|
| Compared with normal group : * p<0.05, ** p<0.01; | | | | |
| compared with model group : □ p<0.05, □□ p<0.01 ; | | | | |
| compared with simple prescription group : # p<0.05, ## p<0.01 . n=7 | | | | |

**Table 17.**

| Effect of lotus root joints compound prescription on the blood sugar, Ins and ISI of nutrition-type obesity rats | | | |
|---|---|---|---|
| X±S | | | |
| Group | Blood sugar (mmol/L) | Ins (Mu/L) | ISI |
| Blank group | 4.38±0.52 | 36.54±5.78 | - 5.142±0.164 |
| Model group | 5.44±0.76 | 69.08±11.33** | - 6.083±0.214** |
| Simple prescription group | 5.03±0.48 | 48.14±15.22^{□□} | - 5.504±0.204*^{□□} |
| Compound prescription 1 group | 5.21±0.83 | 46.57±12.36^{□□} | - 5.493±0.291*^{□□} |
| Compound prescription 2 group | 5.13±0.72 | 47.03±13.34 | - 5.481±0.128*^{□□} |
| Compound prescription 3 group | 4.79±0.55 | 42.35±7.83^{□□} | - 5.301±0.172^{□□} |

| | | | |
|---|---|---|---|
| Compared with normal group : * p<0.05, | | | |
| ** p<0.01; | | | |
| compared with model group : □ p<0.05, □□ p<0.01 . n=7 | | | |

**Table 18.**

| Effect of lotus root joints compound prescription on the blood viscosity of nutrition-type obesity rats | | |
|---|---|---|
| X±S | | |
| Group | Serum viscosity ( cP ) | Hct ( % ) |
| Blank group | 1.32±0.09 | 34.12±2.01 |
| Model group | 2.26±0.17** | 37.85±1.98* |
| Simple prescription group | 1.98±0.34**^{□} | 35.87±2.15 |
| Compound prescription 1 group | 1.35±0.28^{□□}^{##} | 34.35±1.79 |
| Compound prescription 2 group | 1.77±0.21*^{□□} | 35.76±2.43 |
| Compound prescription 3 group | 1.340±0.31 ^{□□}^{##} | 34.19±2.03^{□} |

| | | |
|---|---|---|
| Compared with normal group : * p<0.05, | | |
| ** p<0.01; | | |
| compared with model group : □ p<0.05, | | |
| □□ p<0.01; | | |
| compared with simple prescription group : ## p<0.01 . n=7 | | |

**Table 19.**

| Effect of lotus root joints compound prescription on the SOD and LPO of nutrition-type obesity rats | | |
|---|---|---|
| X±S | | |
| Group | SOD(u/g) | LPO(nmol/ml) |
| Blank group | 17.31±1.94 | 0.69±0.12 |
| Model group | 12.35±2.01** | 2.74±0.45** |
| Simple prescription group | 14.83±1.99^{□} | 1.58±0.37**^{□□} |
| Compound prescription 1 group | 15.02±2.35 | 1.16±0.23^{□□}^{#} |
| Compound prescription 2 group | 16.04±2.51^{□} | 0.98±0.32^{□□}^{##} |
| Compound prescription 3 group | 16.98±1.84^{□□} | 0.81±0.14^{□□}^{##} |

| | | |
|---|---|---|
| Compared with normal group : * p<0.05, ** p<0.01; | | |
| compared with model group : □ p<0.05, | | |
| □□ p<0.01; | | |
| compared with simple prescription group : # p<0.05, | | |
| # # p<0.01. n=7 | | |

## Claims

1. A health-care product, wherein it comprises a powder of or an extract of lotus root joints.

2. A health-care product according to claim 1, **characterized in that** it comprises: an extract of lotus root joints 4 ~ 30 part by weight, a green tea and/or extract thereof 0.08-2 part by weight, and notoginseng and/or extract thereof 0.08-0.5 part by weight.

3. A health-care product according to claim 2, **characterized in that** it contains 4 ~ 7.5 part by weight of the extract of lotus root joints.

4. A health-care product according to claim 2, **characterized in that** it further contains 2 ~ 15 part by weight of a powder of lotus root joints.

5. A health-care product according to claim 1, **characterized in that** the health-care product is formulated in the form of a Tablet, capsule, soluble granule, solution or injection.

6. A process for preparation of a health-care product, comprising the following steps:
a) Pulverizing lotus root joints and thus obtaining a powder of the joints;
b) Putting the powder of the joints in a product-grade solvent for extraction, then filtering it, and using the filtrate solution as the extract of the joints; and
c) Blending conventional product-grade adjuvant with the powder of the joints or the extract of the joints, and then formulating the blend into a form of Tablet, capsule, soluble granule, solution or injection.

7. A process according to claim 6, **characterized in that** the steps a) and b) are carried out by any of the following variants:
( 1 )Drying lotus root joints and pulverizing the dried joints into a powder having a granule size of 20 mesh; putting the powder into a container and adding therein 5 ~ 10 times by weight of 30% ~ 90% ethanol, based on the weight of the powder; extracting it at room temperature for 24 hours, then filtering the mixture; extracting the filtrate residue for another 2 times using the same procedure; combining the filtrate solutions, concentrating the combined filtrate solution under vacuum and freeze-drying it into a dried powder; or
(2) Drying the lotus root joints and pulverizing the dried joints into a powder having a granule size of 20 mesh, putting the powder into a container and adding therein 5 ~ 10 times by weight of 30% ~ 90% ethanol, based on the weight of the powder; heating it in water bath and refluxing for 30 ~ 60 minutes, then filtering it while it is hot; extracting the filtrate residue for another 2 times using the same procedure as mentioned above; combining the filtrate solutions, concentrating it at a low temperature under reduced pressure, and freeze-drying it into a dried powder; or
(3)Drying lotus root joints and pulverizing it into granules of soybean's size; putting it into a container and adding therein 6 ~ 10 times by weight of fresh water, based on the weight of the powder; heating it at its boiling point for 30 ~ 40 minutes, then filtering it while it is hot; extracting the filtrate residue for another 2 times using the same procedure as mentioned above; combining the filtrate solutions, concentrating the combined solution under vacuum and drying it into a powder.

8. A process according to claim 6, **characterized in that** 4 ~ 7.5 part by weight of extract of lotus root joints is prepared according to steps a) and b); and another 0.08-2 part by weight of green tea and/or extract thereof, 0.08-0.5 part by weight of notoginseng and/or extract thereof are added.

9. A process according to claim 8, **characterized in that** further 2 ~ 15 part by weight of powder of lotus root joints is added.

10. Use of the health-care product according to any one of claim 1 to 5 in preparation of following medicines:
Medicine for improving human insulin resistance;
Medicine for treating or preventing obesity;
Medicine for treating or preventing hypertension;
Medicine for treating or preventing hyperlipoidemia;
Medicine for treating or preventing Diabetes Mellitus;
Medicine for improving blood viscosity, preventing thrombosis, and promoting microcirculation;
Medicine for treating or preventing Alzheimer's Disease and against senility.
